# EUROPEAN PATENT SPECIFICATION

(11) **EP 2 965 726 B1**
(45) Date of publication and mention of the grant of the patent: **01.02.2017**
(21) Application number: 15176338.0
(22) Date of filing: 10.07.2015
(51) Int. Cl.: A61F 5/02

(54) **IMPROVED LUMBAR CORSET**
VERBESSERTES LUMBALKORSETT
CORSET LOMBAIRE AMÉLIORÉ

(30) Priority: 11.07.2014 IT MI20141269
(43) Date of publication of application: 13.01.2016
(73) Proprietor: Orthoservice AG, 6830 Chiasso (CH)
(72) Inventor: ROSSI, Paolo, 6834 MORBIO INFERIORE (CH); RAVESE, Mauro, 23825 ESINO LARIO (LC) (IT); TURCONI, Francesco, 20900 MONZA (MB) (IT); BERNAREGGI, Aldo, 20842 BESANA IN BRIANZA (MB) (IT)
(74) Representative: Ottazzo, Marco Francesco Agostino

(56) References cited:
- EP-A1- 2 670 364
- WO-A1-2014/006521
- US-A- 3 927 665

## Description

The present invention generally refers to a lumbar corset and, more in particular, to a closing system for a corset that can be used for the prevention and treatment of diseases of the lumbar-sacral section of the user.

Lumbar corsets are well known orthopaedic devices which allow treating some diseases and disorders of the trunk, such as for example lumbago, lumbar sciatica and lumbar-sacralgia, discopathy, arthrosis, slight trauma of the lumbar column, lumbar paravertebral muscle contractures, disc herniation and much more. Lumbar corsets may also be used in the after-operation treatment of the patient.

A typical lumbar corset is normally constituted by an openable band which is worn on the trunk of a user, in proximity of the lumbar area. The band is usually made of an elastic fabric or non-elastic fabric and it is provided with a dorsal panel, at times reinforced by semi-rigid sticks with protected tips. Some lumbar corset models are provided with flexible sticks, serving for anti-rolling purposes, on the sides and on the abdomen, as well as an adjustable closing system of the adhesive or tear type.

Over time, the lumbar corsets of the known type have been equipped with more and more reliable closure systems which are however complex to make and use. For example, document EP 2 400 936 A1 describes a lumbar corset provided with a closing system slidably mounted on a rigid plate. Thus, the closing system comprises a first flexible coupling accessory and a second flexible coupling accessory, respectively fixable to the two strap elements forming the lumbar band of the corset. The closing system, on which the two coupling accessories of the strap elements are fixed, is slidably mounted on the rigid plate and it can be moved linearly along two apposite grooves obtained on such rigid plate by means of a complex system of pulleys and idlers.

Document EP 2 672 937 A1 describes a closing system for a lumbar corset comprising a first closure component and a second closure component, connected to each other by means of a stretching element. A stretching mechanism provided with a housing for an extensible and retractable wire is mounted on one of the two closure components. The wire is configured to be increasingly wound in its housing, so as to mutually approach the first closure component and the second closure component and, thus, fasten the corset.

Document EP 2 538 898 A1 describes a lumbar corset provided with an adjustment mechanism constituted by two opposite rigid connectors. The two rigid connectors can be respectively fixed to two strap elements forming the lumbar band of the corset and they are provided with a plurality of return pulleys. Such pulleys can be engaged by a pair of ropes which allow adjusting the corset by approaching or moving the two rigid connectors and, thus, the two strap elements, away.

Document EP 2 670 364 A1 describes a lumbar support belt whose adjustment mechanism is constituted by a dorsal strap slidable within fixed buckles and free buckles. In addition, the mechanism comprises a pair of fastening straps respectively fixed, on one side, to the two half-parts forming the lumbar band of the belt and respectively received through the free buckles. The fastening straps, on the side opposite to the one fixed to the two half-parts, are configured to be coupled to such half-parts, thus multiplying the traction force exerted by the adjustment mechanism as a whole.

Document WO 2014/006521 A1 describes a corset comprising a first portion and a second portion, with a first plurality of strips departing from the first portion towards the second portion and a second plurality of strips departing from the second portion towards the first portion. The strips of each plurality are grouped in at least first and second groups, each of which is connected to a respective first and second connection elements. The corset further comprises tensioning elements permanently constrained to a respective corset portion, removably constrained to the other corset portion and active on the connection elements. Pre-tensioning elements are arranged to connect the first and the second connection elements to the respective tensioning elements and they are removably constrainable to the two corset portions.

Thus, an object of the present invention is to provide a lumbar corset and, more in particular, a closing system for such lumbar corset, that is capable of overcoming the aforementioned drawbacks of the prior art in an extremely simple, inexpensive and particularly functional manner.

In detail, an object of the present invention is to provide a lumbar corset provided with a simplified closing system, that is easy to manufacture and use.

Another object of the present invention is to provide a lumbar corset provided with an efficient and safe closing system.

A further object of the present invention is to provide a lumbar corset provided with a closing system without rigid components that are complex, expensive and requiring special moulding procedures for the manufacture thereof.

These objects according to the present invention are achieved by providing a lumbar corset and, more in particular, a closing system for such lumbar corset, as outlined in claim 1.

Further characteristics of the invention are outlined by the dependent claims, which are an integral part of the present description.

The characteristics and advantages of a lumbar corset according to the present invention will be clearer from the following exemplifying and nonlimiting description with reference to the attached schematic drawings wherein:
figure 1 is a schematic view of a first embodiment of the lumbar corset according to the present invention, shown in open and flat configuration and from the outer side thereof;
figure 2 is a schematic view of the lumbar corset of figure 1, shown in closed configuration around the lumbar area of a user and with the respective closing system fastened;
figure 3 is a schematic view of the lumbar corset of figure 1, shown in closed configuration around the lumbar area of a user and with the respective closing system partly open or released;
figure 4 is a schematic view of the lumbar corset of figure 1, in which there are observed (indicated with corresponding arrows) the directions of movement of the single straps of the closing system during the step of closing the corset;
figure 5 is a schematic view of a second embodiment of the lumbar corset according to the present invention, shown in open and flat configuration and from the outer side thereof;
figure 6 is a schematic view of the lumbar corset of figure 5, shown in closed configuration around the lumbar area of a user and with the respective closing system fastened;
figure 7 is a schematic view of the lumbar corset of figure 5, shown in closed configuration around the lumbar area of a user and with the respective closing system partly open or released; and
figure 8 is a schematic view of a third embodiment of the lumbar corset according to the present invention.

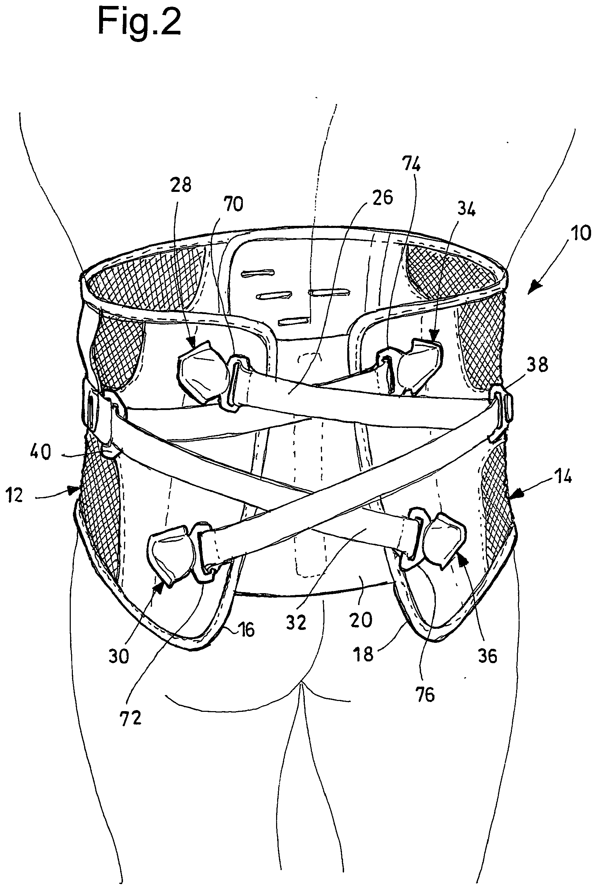

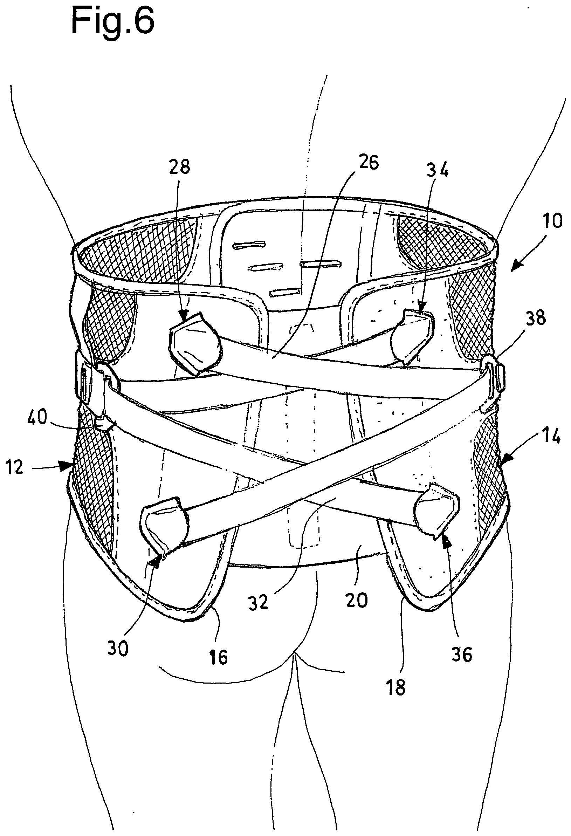

With reference to the figures, some preferred embodiments of the lumbar corset according to the present invention are shown, indicated in their entirety with reference number 10. The lumbar corset 10 is constituted by a plurality of mutually assembled parts which allow the lumbar corset 10 to be easily worn and to maintain a correct posture of the lumbar area of a user after being adjusted and fastened around the waist (figures 2 and 6).

In detail, the lumbar corset 10 is constituted by an openable band obtained by joining two half-parts 12 and 14. Each of the two half-parts 12 and 14 of the openable band is preferably made of a transpiring rigid fabric and it can be internally provided with padding elements and/or one or more rigid or semi-rigid sticks.

The two half-parts 12 and 14 of the openable band are permanently joined, at the respective distal or dorsal ends 16 and 18, by means of a dorsal joining member 20. The dorsal joining member 20 is preferably obtained as a band made of an elastic fabric, but it could also be more simply constituted by a seam joining the distal ends 16 and 18 of the two half-parts 12 and 14. The respective proximal or abdominal ends 22 and 24 of the two half-parts 12 and 14 are instead configured to overlap each other and be joined in the closed configuration of the lumbar corset 10, i.e. when such lumbar corset 10 is wrapped around the lumbar area of a user.

The lumbar corset 10 is provided with a closing system constituted by a set of straps applied on the outer side of the lumbar corset 10. The purpose of such set of straps is to hold the two half-parts 12 and 14 of the openable band to each other and facilitate the wrapping action of the lumbar corset 10, as well as guarantee a correct, easy and quick positioning of the lumbar corset 10 around the lumbar area of a user.

In detail, the set of straps comprises at least one first dorsal strap 26, constrained to the distal end 16 of a first half-part 12 at a first upper constraint point 28 and a first lower constraint point 30 arranged along a first axis substantially perpendicular to the longitudinal direction of development of the openable band forming the lumbar corset 10. The set of straps also comprises at least one second dorsal strap 32, constrained to the distal end 18 of the second half-part 14 at a second upper constraint point 34 and a second lower constraint point 36, also arranged along a second axis substantially perpendicular to the longitudinal direction of development of the openable band forming the lumbar corset 10. The presence of two distinct dorsal straps 26 and 32, at least one for each of the two half-parts 12 and 14 forming the openable band of the lumbar corset 10, allows an efficient and independent adjustment for each of such two half-parts 12 and 14, contrary to corsets of the known type, such as for example the one described in document EP 2 670 364 A1, where the presence of single crossed dorsal strap does not allow a separate adjustment of the two half-parts once the corset is worn around the lumbar area of a user.

Starting from the respective upper 28 and lower 30 constraint points, the first dorsal strap 26 extends longitudinally towards the proximal end 24 of the second half-part 14 in order to wrap around a first free pulley 38, i.e. not directly fixed on such second half-part 14. Analogously, starting from the respective upper 34 and lower 36 constraint points, the second dorsal strap 32 extends longitudinally towards the proximal end 22 of the first half-part 12 in order to wrap around a second free pulley 40, i.e. not directly fixed on such first half-part 12.

The set of straps further comprises a first primary side strap 42, made integral with the first half-part 12 at a first predefined fixing point 44, and a second primary side strap 46, made integral with the second half-part 14 at a second predefined fixing point 48. Both predefined fixing points 44 and 48 are preferably arranged along the same longitudinal axis, with reference to the direction of development of the openable band forming the lumbar corset 10.

Both the primary side straps 42 and 46 are provided, at the respective free ends, with respective releasable anchoring means 50 and 52, couplable with respective anchoring portions 54 and 56 provided at the proximal ends 22 and 24 of the two half-parts 12 and 14. Preferably, the releasable anchoring means 50 and 52 are couplable with the respective anchoring portions 54 and 56 by means of a Velcro®-type system, using micro-hooks obtained on the releasable anchoring means 50 and 52 and with micro-slots obtained on at least part of the surface of each coupling portion 54 and 56.

The first primary side strap 42 is operatively connected to the corresponding second dorsal strap 32 by interposing, in addition to the second free pulley 40, of a first mobile pulley 58 and a first secondary side strap 60, said first secondary side strap 60 being made integral with the first half-part 12 at a first predefined fixing point 62. Analogously, the second primary side strap 46 is operatively connected to the corresponding first dorsal strap 26 by interposing, in addition to the first free pulley 38, of a second mobile pulley 64 and a second secondary side strap 66, said second secondary side strap 66 being made integral with the second half-part 14 at a second predefined fixing point 68.

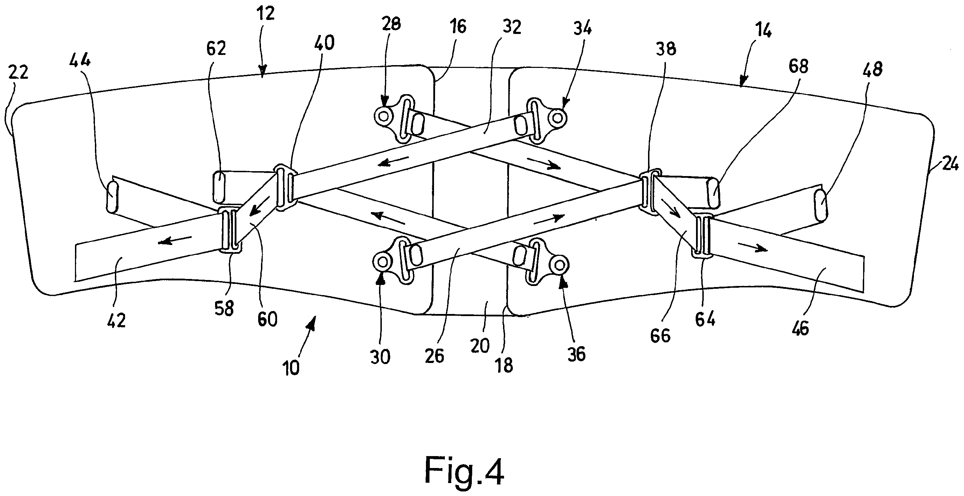

Advantageously, the second free pulley 40 and the first mobile pulley 58 of the first half-part 12, as well as the first free pulley 38 and the second mobile pulley 64 of the second half-part 14, are arranged along a same axis parallel to the longitudinal direction of development of the openable band forming the lumbar corset 10. This configuration of the set of straps forming the closing system of the lumbar corset 10, with all pulleys arranged along the same axis, allows obtaining greater efficiency in the system of traction forces with respect to the case where such pulleys are offset. Actually, in case of offset pulleys, part of the force is dissipated in the vertical direction or, in other words, in the direction orthogonal to the longitudinal direction of development of the openable band forming the lumbar corset 10. This configuration of the set of straps forming the closing system of the lumbar corset 10 further allows multiplying the traction and closure forces, exerted on the dorsal straps 26 and 32 through the primary side straps 42 and 46, in the closed configuration of the lumbar corset 10 (figure 4). A similar configuration of the set of straps forming the closing system of the lumbar corset 10 also allows simplifying the traction movement, exerted on the releasable anchoring means 50 and 52 of the primary side straps 42 and 46, required to obtain the adjustment and closing of the lumbar corset 10.

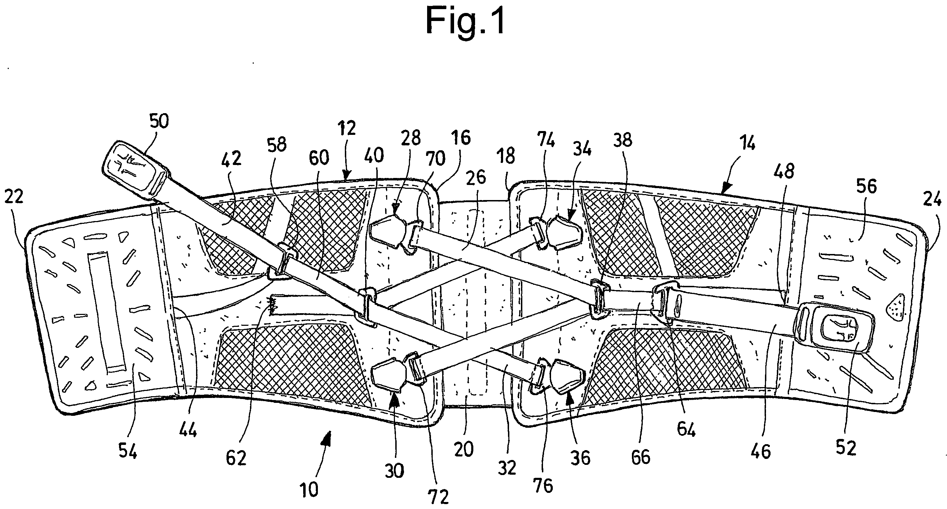

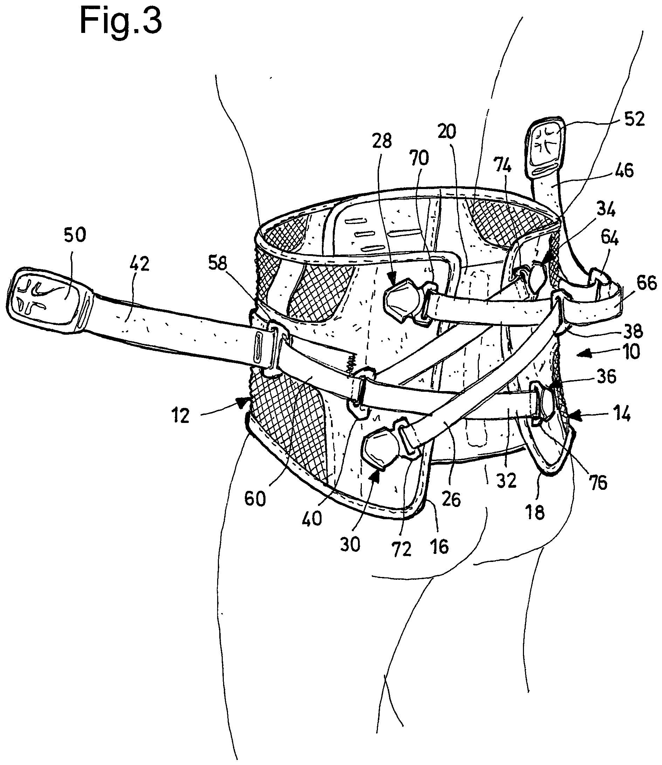

With reference to figures 1 to 4, it can be observed that in the first embodiment of the lumbar corset 10 the first dorsal strap 26 is constrained to the distal end 16 of the first half-part 12 through a first pair of rotating anchors 70 and 72 respectively hinged at the first upper constraint point 28 and the first lower constraint point 30. Analogously, the second dorsal strap 32 is constrained to the distal end 18 of the second half-part 14 through a second pair of rotating anchors 74 and 76 respectively hinged at the second upper constraint point 34 and the second lower constraint point 36. The two pairs of rotating anchors 70, 72 and 74, 76, being capable of performing relative rotations around the respective hinging points, allow varying the directions of development of the respective dorsal straps 26 and 32 in the steps of closing the lumbar corset 10, to the advantage of the wearability of such lumbar corset 10 by the user.

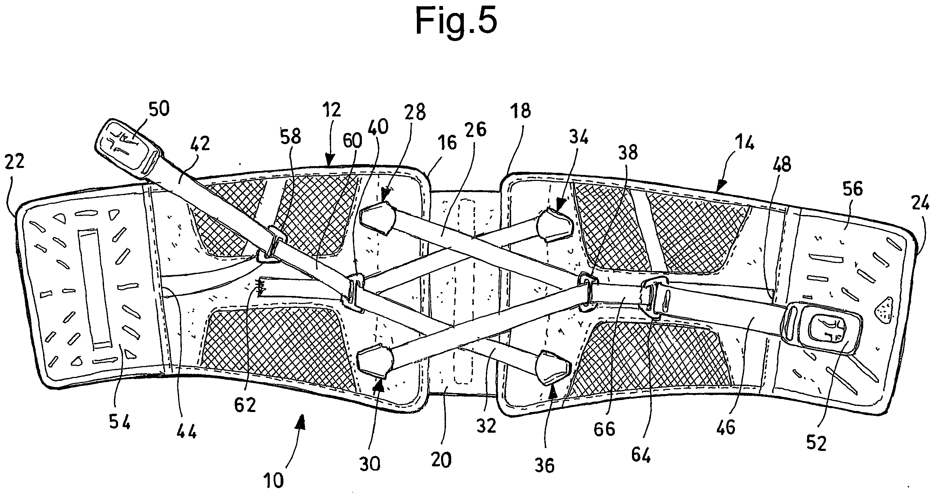

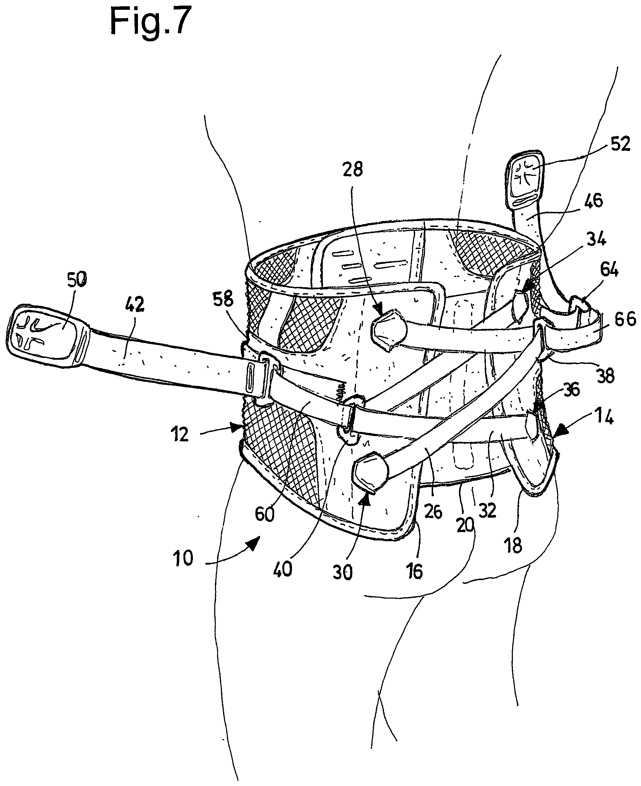

Now, with reference to figures 5 to 7, it is observed that in the second embodiment of the lumbar corset 10 the first dorsal strap 26 is unreleasably fixed at the first upper constraint point 28 and the first lower constraint point 30, for example by means of a seam. Analogously, also the second dorsal strap 32 is unreleasably fixed at the second upper constraint point 34 and the second lower constraint point 36, for example by means of a seam. The seams, which maintain the dorsal straps 26 and 32 stably fixed to the respective distal ends 16 and 18 of the two half-parts 12 and 14, are designed so as to impart to such dorsal straps 26 and 32 respective predefined directions of development with the aim of obtaining a correct closure of the lumbar corset 10.

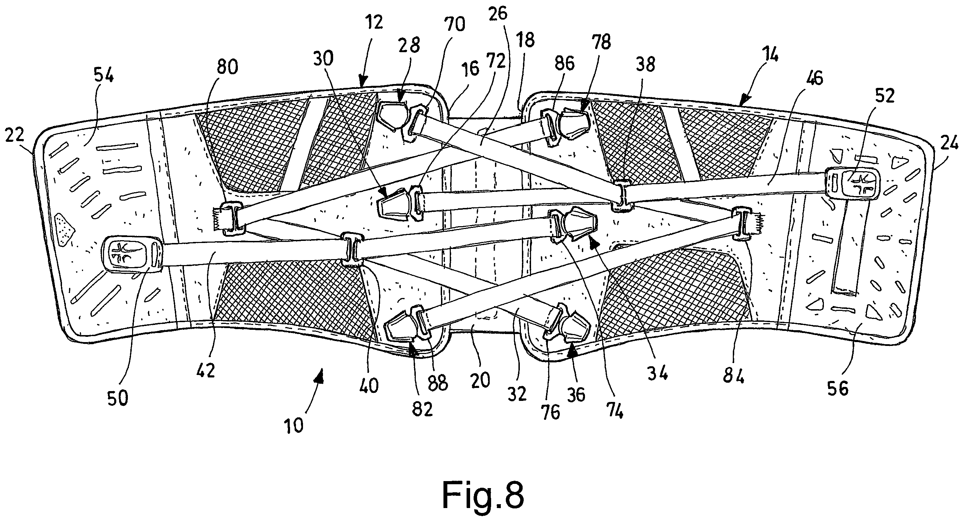

Lastly, with reference to figure 8, a third embodiment of the lumbar corset 10 is shown. In this third embodiment, the dorsal straps 26 and 32 are directly connected to the respective primary side straps 42 and 46 by the interposition of the respective first free pulley 38 and second free pulley 40.

In detail, the first primary side strap 42 is constituted by a crossed strap provided, in sequence, with a first constraint point 78 on the distal end 18 of the second half-part 14, a first diverting pulley 80 constrained to the first half-part 12 and a second diverting pulley coinciding with the second free pulley 40. Thus, the first primary side strap 42 terminates with a first releasable anchoring means 50 which can be coupled with the respective coupling portion 54 provided at the proximal end 22 of the first half-part 12.

Analogously, also the second primary side strap 46 is constituted by a crossed strap provided, in sequence, with a first constraint point 82 on the distal end 16 of the first half-part 12, a first diverting pulley 84 constrained to the second half-part 14 and a second diverting pulley coinciding with the first free pulley 38. Thus, the second primary side strap 46 terminates with a second releasable anchoring means 52 which can be coupled with the respective coupling portion 56 provided at the proximal end 24 of the second half-part 14.

In this third embodiment, the dorsal straps 26 and 32 are preferably constrained to the respective distal ends 16 and 18 of the two half-parts 12 and 14 through respective pairs of rotating anchors 70, 72 and 74, 76 respectively hinged at the first upper constraint points 28 and 34 and the first lower constraint points 30 and 36. In the same manner, the first primary side strap 42 and the second primary side strap 46 are preferably respectively constrained to the distal end 18 of the second half-part 14 and to the distal end 16 of the first half-part 12 through rotating anchors 86 and 88 respectively hinged at the first constraint points 78 and 82. Additionally, in this third embodiment the second diverting pulleys, coinciding with the first free pulley 38 and with the second free pulley 40, are arranged offset with respect to the longitudinal development axis of the openable band forming the lumbar corset 10.

It has thus been observed that the lumbar corset according to the present invention achieves the previously outlined objectives.

Contrary to the prior art solutions which provide for the use of plates or other rigid components, the lumbar corset according to the present invention offers high comfort for the lumbar area, in that almost all respective components are made of fabric, to the advantage of wearability for the user. Simultaneously, the flexible material the openable band is made of always guarantees the correct adherence of the lumbar corset to the anatomic shape of the user, also offering the support and compression required for maximum adherence and best comfort. Lastly, set of straps and pulleys guarantee correct, easy and fast positioning of the lumbar corset both while worn and during the subsequent adjustment steps.

The lumbar corset of the present invention thus conceived is susceptible to numerous modifications and variants, all falling within the same inventive concept; in addition, all details can be replaced by technically equivalent elements. In practice, the materials used, as well as the shapes and dimensions, may vary according to the technical requirements.

The scope of protection of the invention is thus defined by the attached claims.

## Claims

1. Lumbar corset (10) consisting of a band which can be opened, made by joining two permanently joined half-parts (12, 14), at the respective distal ends (16, 18), by means of a joining member (20), the respective proximal ends (22, 24) of said two half-parts (12, 14) being instead designed to overlap each other and be joined in the lumbar corset (10) closed configuration, said lumbar corset (10) being provided with a closing system consisting of an assembly made of straps applied on the outer side of said lumbar corset (10) and comprising:
- at least a first dorsal strap (26), constrained to the distal end (16) of a first half-part (12) at a first upper constraint point (28) and at a first lower constraint point (30), wherein said first dorsal strap (26) extends longitudinally towards the proximal end (24) of the second half-part (14) in order to wrap around a first free pulley (38);
- at least a second dorsal strap (32), constrained to the distal end (18) of the second half-part (14) at a second upper constraint point (34) and at a second lower constraint point (36), wherein said second dorsal strap (32) extends longitudinally towards the proximal end (22) of the first half-part (12) in order to wrap around a second free pulley (40);
- a first primary side strap (42), operatively connected to the second dorsal strap (32) through said second pulley (40) which is free and made integral with the first half-part (12) at a first predefined fixing point (44; 80); and
- a second primary side strap (46), operatively connected to the first dorsal strap (26) through said first pulley (38) which is free and made integral with the second half-part (14) at a second predefined fixing point (48; 84),
wherein both primary side straps (42, 46) are provided, at their respective free ends, with respective releasable anchoring means (50, 52), which can be coupled with respective anchoring portions (54, 56) provided at said proximal ends (22, 24), **characterized in that** the first primary side strap (42) is operatively connected to the corresponding second dorsal strap (32) by interposing, in addition to said second free pulley (40), a first mobile pulley (58) and a first secondary side strap (60), said first secondary side strap (60) being made integral with the first half-part (12) at a first predefined fixing point (62), **and in that** the second primary side strap (46) is operatively connected to the corresponding first dorsal strap (26) by interposing, in addition to said first free pulley (38), a second mobile pulley (64) and a second secondary side strap (66), said second secondary side strap (66) being made integral with the second half-part (14) at a second predefined fixing point (68), **and in that** said second free pulley (40) and said first mobile pulley (58) of the first half-part (12), as well as said first free pulley (38) and said second mobile pulley (64) of the second half-part (14), are arranged along a same axis which is parallel to the longitudinal development direction of the openable band forming the lumbar corset (10).

2. Lumbar corset (10) according to claim 1, **characterized in that** said first upper constraint point (28) and first lower constraint point (30) are arranged along a first axis, substantially perpendicular to the longitudinal development direction of the openable band forming the lumbar corset (10), and **in that** said second upper constraint point (34) and second lower constraint point (36) are arranged along a second axis, also substantially perpendicular to the longitudinal development direction of the openable band forming the lumbar corset (10).

3. Lumbar corset (10) according to claim 1 or 2, **characterized in that** said first predefined fixing point (44; 80) and said second predefined fixing point (48; 84) are arranged along a same longitudinal axis, with respect to the development direction of the openable band forming the lumbar corset (10).

4. Lumbar corset (10) according to any claim 1 to 3, **characterized in that** the first dorsal strap (26) is constrained to the distal end (16) of the first half-part (12) through a first pair of rotating anchors (70, 72), hinged at the first upper (28) and lower (30) constraint points, respectively, and **in that** the second dorsal strap (32) is constrained to the distal end (18) of the second half-part (14) through a second pair of rotating anchors (74, 76), hinged at the second upper (34) and lower (36) constraint points, respectively, said two pairs of rotating anchors (70, 72; 74, 76) being able to carry out relative rotations about the respective hinging points in order to make the change of the development directions of the respective dorsal straps (26, 32) in the closing steps of the lumbar corset (10) possible.

5. Lumbar corset (10) according to any claim 1 to 3, **characterized in that** the first dorsal strap (26) is unreleasably attached at the first upper contraint point (28) and at the first lower contraint point (30), whereas the second dorsal strap (32) is unreleasably attached at the second upper contraint point (34) and at the second lower constraint point (36), so as to impose to said dorsal straps (26, 32) respective predefined development directions in order to achieve a correct closing of the lumbar corset (10).

6. Lumbar corset (10) according to any claim 1 to 5, **characterized in that** the releasable anchoring means (50, 52) can be coupled with the respective anchoring portions (54, 56) by means of a Velcro^{®}-type system.

7. Lumbar corset (10) according to any claim 1 to 6, **characterized in that** the joining member (20) is made in the form of a tape made of elastic fabric.

8. Lumbar corset (10) according to any claim 1 to 7, **characterized in that** each of the two half-parts (12, 14) of the openable band is made of transpiring fabric.

9. Lumbar corset (10) according to any claim 1 to 8, **characterized in that** each of the two half-parts (12, 14) of the openable band is internally provided with padding elements.

10. Lumbar corset (10) according to any claim 1 to 9, **characterized in that** each of the two half-parts (12, 14) of the openable band is internally provided with one or more rigid or semi-rigid sticks.

## Patentansprüche

1. Lumbalkorsett (10), bestehend aus einer Binde, die geöffnet werden kann, hergestellt durch Verbinden zweier dauerhaft verbundener Halbteile (12, 14) an ihren jeweiligen distalen Enden (16, 18) mittels eines Verbindungsgliedes (20), wobei die jeweiligen proximalen Enden (22, 24) der beiden Halbteile (12, 14) hingegen ausgestaltet sind, um sich gegenseitig zu überlappen und in der geschlossenen Ausgestaltung des Lumbalkorsetts (10) verbunden zu werden, wobei das Lumbalkorsett (10) mit einem Verschlusssystem bestehend aus einer Gruppe von Gurten versehen ist, die auf der Außenseite des Lumbalkorsetts (10) angebracht werden und Folgendes umfassen:
- wenigstens einen ersten dorsalen Gurt (26), der an dem distalen Ende (16) eines ersten Halbteils (12) in einem ersten oberen Einspannpunkt (28) und in einem ersten unteren Einspannpunkt (30) festgelegt ist, wobei der erste dorsale Gurt (26) sich längs zum proximalen Ende (24) des zweiten Halbteils (14) hin erstreckt, um eine erste lose Rolle (38) zu umschlingen;
- wenigstens einen zweiten dorsalen Gurt (32), der an dem distalen Ende (18) des zweiten Halbteils (14) in einem zweiten oberen Einspannpunkt (34) und in einem zweiten unteren Einspannpunkt (36) festgelegt ist, wobei der zweite dorsale Gurt (32) sich längs zum proximalen Ende (22) des ersten Halbteils (12) hin erstreckt, um eine zweite lose Rolle (40) zu umschlingen;
- einen ersten Hauptseitengurt (42), der mit dem zweiten dorsalen Gurt (32) durch die zweite Rolle (40) wirkverbunden ist, die lose und mit dem ersten Halbteil (12) in einem ersten vordefinierten Befestigungspunkt (44; 80) einstückig ausgebildet ist; und
- einen zweiten Hauptseitengurt (46), der mit dem ersten dorsalen Gurt (26) durch die erste Rolle (38) wirkverbunden ist, die lose und mit dem zweiten Halbteil (14) in einem zweiten vordefinierten Befestigungspunkt (48; 84) einstückig ausgebildet ist,
wobei beide Hauptseitengurte (42, 46) an ihren jeweiligen freien Enden jeweils mit lösbaren Verankerungsmitteln (50, 52) versehen sind, die mit jeweiligen Verankerungsabschnitten (54, 56) verbunden werden können, die an den proximalen Enden (22, 24) vorgesehen sind, **dadurch gekennzeichnet, dass** der erste Hauptseitengurt (42) mit dem entsprechenden zweiten dorsalen Gurt (32) wirkverbunden ist, indem zusätzlich zu der zweiten losen Rolle (40) eine erste bewegliche Rolle (58) und ein erster Nebenseitengurt (60) eingefügt werden, wobei der erste Nebenseitengurt (60) mit dem ersten Halbteil (12) in einem ersten vordefinierten Befestigungspunkt (62) einstückig ausgebildet ist, **und dass** der zweite Hauptseitengurt (46) mit dem entsprechenden ersten dorsalen Gurt (26) wirkverbunden ist, indem zusätzlich zu der ersten losen Rolle (38) eine zweite bewegliche Rolle (64) und ein zweiter Nebenseitengurt (66) eingefügt sind, wobei der zweite Nebenseitengurt (66) einstückig mit dem zweiten Halbteil (14) in einem zweiten vordefinierten Befestigungspunkt (68) ausgebildet ist, **und dass** die zweite lose Rolle (40) und die erste bewegliche Rolle (58) des ersten Halbteils (12), ebenso wie die erste lose Rolle (38) und die zweite bewegliche Rolle (64) des zweiten Halbteils (14) längs derselben Achse angeordnet sind, die parallel zur Längsabwicklungsrichtung der zu öffnenden, das Lumbalkorsett (10) bildenden Binde verläuft.

2. Lumbalkorsett (10) nach Anspruch 1, **dadurch gekennzeichnet, dass** der erste obere Einspannpunkt (28) und der erste untere Einspannpunkt (30) längs einer ersten Achse angeordnet sind, die im Wesentlichen senkrecht zur Längsabwicklungsrichtung der zu öffnenden, das Lumbalkorsett (10) bildenden Binde verläuft, und dass der zweite obere Einspannpunkt (34) und der zweite untere Einspannpunkt (36) längs einer zweiten Achse angeordnet sind, die ebenfalls im Wesentlichen senkrecht zur Längsabwicklungsrichtung der zu öffnenden, das Lumbalkorsett (10) bildenden Binde verläuft.

3. Lumbalkorsett (10) nach Anspruch 1 oder 2, **dadurch gekennzeichnet, dass** der erste vordefinierte Befestigungspunkt (44; 80) und der zweite vordefinierte Befestigungspunkt (48; 84) längs einer mit Bezug auf die Abwicklungsrichtung der zu öffnenden, das Lumbalkorsett (10) bildenden Binde gleichen Längsachse angeordnet sind.

4. Lumbalkorsett (10) nach einem der Ansprüche 1 bis 3, **dadurch gekennzeichnet, dass** der erste dorsale Gurt (26) an dem distalen Ende (16) des ersten Halbteils (12) durch ein erstes Paar von rotierenden Ankern (70, 72) festgelegt ist, die jeweils an dem ersten oberen (28) und unteren (30) Einspannpunkt angelenkt sind, und dass der zweite dorsale Gurt (32) an dem distalen Ende (18) des zweiten Halbteils (14) durch ein zweites Paar von rotierenden Ankern (74, 76) festgelegt ist, die jeweils an dem zweiten oberen (34) und unteren (36) Einspannpunkt angelenkt sind, wobei die zwei Paare von rotierenden Ankern (70, 72; 74, 76) in der Lage sind, jeweilige Drehungen um die entsprechenden Anlenkpunkte auszuführen, um einen Wechsel der Abwicklungsrichtungen der jeweiligen dorsalen Gurte (26, 32) in den Schritten zum Verschließen des Lumbalkorsetts (10) zu ermöglichen.

5. Lumbalkorsett (10) nach einem der Ansprüche 1 bis 3, **dadurch gekennzeichnet, dass** der erste dorsale Gurt (26) unlösbar an dem ersten oberen Einspannpunkt (28) und an dem ersten unteren Einspannpunkt (30) befestigt ist, während der zweite dorsale Gurt (32) unlösbar an dem zweiten oberen Einspannpunkt (34) und an dem zweiten unteren Einspannpunkt (36) befestigt ist, derart, dass den dorsalen Gurten (26, 32) jeweilige vordefinierte Abwicklungsrichtungen auferlegt werden, um ein korrektes Verschließen des Lumbalkorsetts (10) zu erreichen.

6. Lumbalkorsett (10) nach einem der Ansprüche 1 bis 5, **dadurch gekennzeichnet, dass** die lösbaren Verankerungsmittel (50, 52) mittels eines Klettbandsystems mit den jeweiligen Verankerungsabschnitten (54, 56) verbunden werden können.

7. Lumbalkorsett (10) nach einem der Ansprüche 1 bis 6, **dadurch gekennzeichnet, dass** das Verbindungsglied (20) in der Form eines Bandes aus elastischem Gewebe hergestellt ist.

8. Lumbalkorsett (10) nach einem der Ansprüche 1 bis 7, **dadurch gekennzeichnet, dass** jedes der beiden Halbteile (12, 14) der zu öffnenden Binde aus einem atmungsaktiven Gewebe hergestellt ist.

9. Lumbalkorsett (10) nach einem der Ansprüche 1 bis 8, **dadurch gekennzeichnet, dass** jedes der beiden Halbteile (12, 14) der zu öffnenden Binde inwendig mit Polsterelementen versehen ist.

10. Lumbalkorsett (10) nach einem der Ansprüche 1 bis 9, **dadurch gekennzeichnet, dass** jedes der beiden Halbteile (12, 14) der zu öffnenden Binde inwendig mit einem oder mehreren steifen oder halbsteifen Stäben versehen ist.

## Revendications

1. Corset lombaire (10) constitué d'une bande qui peut être ouverte, réalisée en joignant deux moitiés reliées de manière permanente (12, 14), au niveau d'extrémités distales respectives (16, 18), au moyen d'un élément d'assemblage (20), les extrémités proximales respectives (22, 24) desdits deux moitiés (12, 14) étant au contraire conçues pour se chevaucher entre elles et être reliées dans la configuration fermée du corset lombaire (10), ledit corset lombaire (10) étant muni d'un système de fermeture comprenant un ensemble constitué de sangles appliquées sur le côté extérieur dudit corset lombaire (10) et comprenant :
- au moins une première sangle dorsale (26), liée à l'extrémité distale (16) d'une première moitié (12) au niveau d'un premier point de liaison supérieur (28) et au niveau d'un premier point de liaison inférieur (30), dans lequel ladite première sangle dorsale (26) s'étend longitudinalement vers l'extrémité proximale (24) de la deuxième moitié (14) de manière à s'enrouler autour d'une première poulie libre (38) ;
- au moins une deuxième sangle dorsale (32), liée à l'extrémité distale (18) d'une deuxième moitié (14) au niveau d'un deuxième point de liaison supérieur (34) et au niveau d'un deuxième point de liaison inférieur (36), dans lequel ladite deuxième sangle dorsale (32) s'étend longitudinalement vers l'extrémité proximale (22) de la première moitié (12) de manière à s'enrouler autour d'une deuxième poulie libre (40) ;
- une première sangle latérale principale (42), connectée fonctionnellement à la deuxième sangle dorsale (32) par l'intermédiaire de ladite deuxième poulie (40) qui est libre et rendue solidaire de la première moitié (12) au niveau d'un premier point de fixation prédéfini (44 ; 80) ; et
- une deuxième sangle latérale principale (46), connectée fonctionnellement à la première sangle dorsale (26) par l'intermédiaire de ladite première poulie (38) qui est libre et rendue solidaire de la deuxième moitié (14) au niveau d'un deuxième point de fixation prédéfini (48 ; 84),
dans lequel les deux sangles latérales principales (42, 46) sont munies, à leurs extrémités libres respectives, de moyens d'ancrage amovibles respectifs (50, 52), qui peuvent être accouplés avec des parties d'ancrage (54, 56) respectives disposées auxdites extrémités proximales (22, 24), **caractérisé en ce que** la première sangle latérale principale (42) est connectée fonctionnellement à la deuxième sangle dorsale (32) correspondante en interposant, en plus de ladite deuxième poulie libre (40), une première poulie mobile (58) et une première sangle latérale auxiliaire (60), ladite première sangle latérale auxiliaire (60) étant rendue solidaire de la première moitié (12) au niveau d'un premier point de fixation prédéfini (62), et **en ce que** la deuxième sangle latérale principale (46) est connectée fonctionnellement à la première sangle dorsale (26) correspondante en interposant, en plus de ladite première poulie libre (38), une deuxième poulie mobile (64) et une deuxième sangle latérale auxiliaire (66), ladite deuxième sangle latérale auxiliaire (66) étant rendue solidaire de la deuxième moitié (14) au niveau d'un deuxième point de fixation prédéfini (68), et **en ce que** ladite deuxième poulie libre (40) et ladite première poulie mobile (58) de la première moitié (12), ainsi que ladite première poulie libre (38) et ladite deuxième poulie mobile (64) de la deuxième moitié (14), sont agencées le long d'un même axe qui est parallèle à la direction de développement longitudinal de la bande ouvrable formant le corset lombaire (10).

2. Corset lombaire (10) selon la revendication 1, **caractérisé en ce que** ledit premier point de liaison supérieur (28) et ledit premier point de liaison inférieur (30) sont agencés le long d'un premier axe, sensiblement perpendiculaire à la direction de développement longitudinal de la bande ouvrable formant le corset lombaire (10) et **en ce que** ledit deuxième point de liaison supérieur (34) et ledit deuxième point de liaison inférieur (36) sont agencés le long d'un deuxième axe, également sensiblement perpendiculaire à la direction de développement longitudinal de la bande ouvrable formant le corset lombaire (10).

3. Corset lombaire (10) selon la revendication 1 ou 2, **caractérisé en ce que** ledit premier point de fixation prédéfini (44 ; 80) et ledit deuxième point de fixation prédéfini (48 ; 84) sont agencés le long d'un même axe longitudinal, par rapport à la direction de développement de la bande ouvrable formant le corset lombaire (10).

4. Corset lombaire (10) selon l'une quelconque des revendications 1 à 3, **caractérisé en ce que** la première sangle dorsale (26) est liée à l'extrémité distale (16) de la première moitié (12) par l'intermédiaire d'une première paire d'ancrages tournants (70, 72), articulées respectivement aux premiers points de liaison supérieur (28) et inférieur (30), et **en ce que** la deuxième sangle dorsale (32) est liée à l'extrémité distale (18) de la deuxième moitié (14) par l'intermédiaire d'une deuxième paire d'ancrages tournants (74, 76), articulées respectivement aux deuxièmes points de liaison supérieur (34) et inférieur (36), lesdites deux paires d'ancrages tournants (70, 72 ; 74, 76) étant capables d'exécuter des rotations relatives autour des points d'articulation respectifs de manière à effectuer le changement des directions de développement des sangles dorsales (26, 32) respectives dans les étapes de fermeture possibles du corset lombaire (10).

5. Corset lombaire (10) selon l'une quelconque des revendications 1 à 3, **caractérisé en ce que** la première sangle dorsale (26) est attachée de manière inamovible au niveau du premier point de liaison supérieur (28) et au niveau du premier point de liaison inférieur (30), alors que la deuxième sangle dorsale (32) est attachée de manière inamovible au niveau du deuxième point de liaison supérieur (34) et au niveau du deuxième point de liaison inférieur (36), de manière à imposer auxdites sangles dorsales (26, 32) des directions de développement prédéfinies respectives de manière à obtenir une fermeture correcte du corset lombaire (10).

6. Corset lombaire (10) selon l'une quelconque des revendications 1 à 5, **caractérisé en ce que** les moyens d'ancrage amovibles (50, 52) peuvent être accouplés avec les parties d'ancrage (54, 56) respectives au moyen d'un système de type Velcro®.

7. Corset lombaire (10) selon l'une quelconque des revendications 1 à 6, **caractérisé en ce que** l'élément d'assemblage (20) est réalisé sous la forme d'un ruban constitué d'un tissu élastique.

8. Corset lombaire (10) selon l'une quelconque des revendications 1 à 7, **caractérisé en ce que** chacune des deux moitiés (12, 14) de la bande ouvrable est constituée d'un tissu transpirant.

9. Corset lombaire (10) selon l'une quelconque des revendications 1 à 8, **caractérisé en ce que** chacune des deux moitiés (12, 14) de la bande ouvrable est munie intérieurement d'éléments de rembourrage.

10. Corset lombaire (10) selon l'une quelconque des revendications 1 à 9, **caractérisé en ce que** chacune des deux moitiés (12, 14) de la bande ouvrable est munie intérieurement d'une ou plusieurs languettes rigides ou semi-rigides.
